# EUROPÄISCHE PATENTSCHRIFT

(11) **EP 1 138 390 B1**
(45) Veröffentlichungstag und Bekanntmachung des Hinweises auf die Patenterteilung: **06.05.2004**
(21) Anmeldenummer: 01105923.5
(22) Anmeldetag: 09.03.2001
(51) Int. Cl.: B01L 9/06, A61M 5/00

(54) **Transportvorrichtung für medizinische Behälter**
Transporting device for medical containers
Dispositif de transport pour récipients médicaux

(30) Priorität: 15.03.2000 DE 10012575
(43) Veröffentlichungstag der Anmeldung: 04.10.2001
(73) Patentinhaber: Schott Glas, 55122 Mainz (DE); CARL-ZEISS-STIFTUNG trading as SCHOTT GLAS, 55122 Mainz (DE)
(72) Erfinder: Heinz, Jochen, Dr., 55578 Vendersheim (DE); Spallek, Michael, Dr., 55218 Ingelheim (DE); Fabian, Arthur, 55131 Mainz (DE)
(74) Vertreter: Fuchs Mehler Weiss & Fritzsche

(56) Entgegenhaltungen:
- EP-A- 0 790 063
- EP-A- 0 976 453
- WO-A-94/14484
- DE-A- 3 613 489
- DE-A- 4 243 786
- DE-C- 4 021 836
- US-A- 3 643 812

## Beschreibung

Die Erfindung bezieht sich auf eine Transportvorrichtung für Packmittel für medizinisch wirksame Substanzen in Form einer biegesteifen Trägerplatte aus Kunststoff, die eine Vielzahl von Öffnungen zur Aufnahme der Packmittel aufweist.

Primär-Packmittel für medizinisch wirksame Substanzen, wie beispielsweise Fläschchen, Flaschen, Ampullen, Spritzampullen, Glaszylinder, Spritzenkörper, vorgefüllte Spritzen (Fertigspritzen) aus Glas oder Kunststoff, Karpulen, sind in vielfältigen Ausführungsformen bekannt und auf dem Markt.

Diese Primär-Packmittel besitzen typischerweise einen kreisförmigen Querschnitt. Sie können jedoch auch eine mehreckige Querschnittsfläche aufweisen.

Derartige medizinische Primär-Packmittel werden typischerweise zunächst in einem Glas bzw. Kunststoff verarbeitenden Betrieb hergestellt und danach zu einem pharmazeutischen Betrieb transportiert, wo sie befüllt werden. Sowohl bei der Herstellung der Packmittel als auch bei ihrer Befüllung sind eine Reihe von Bearbeitungsschritten notwendig, wie Waschen, Sterilisieren, Verpacken, Transportieren, Silikonisieren, Konfektionieren etc.

Besondere Probleme bereitet hierbei die Handhabung, der Transport und die Lagerung von solchen medizinischen Packmitteln, wie befüllten oder unbefüllten Spritzen, Spritzenkörpern, Zylinderampullen etc. Ein wesentlicher Grund liegt darin, daß diese Packmittel einen Massenartikel darstellen und daher insoweit nicht einzeln gehandhabt werden können, sondern aus wirtschaftlichen Gründen sozusagen nur in geordneten Chargen bzw. magaziniert in sogenannten Trays maschinell gehandhabt werden können.

Entsprechende Vorrichtungen sind in zahlreichen Varianten bekannt geworden. So zeigt die DE 42 43 786 A 1 eine Einrichtung zum Transportieren von Gegenständen, insbesondere von aus Glas, Kunststoff oder dergleichen bestehenden Röhrchen, z.B. Zylindern für Spritzen, Fertigspritzen oder dergleichen mit einem kreisförmigen, umlaufenden Träger, der entlang seines Außenrandes in Abständen aufeinanderfolgende, nach außen offene Aufnahmen für die einzelnen Gegenstände aufweist, die darin aufnehmbar und vom Träger mitnehmbar sind, wobei jede Aufnahme oder jedes Aufnahme-Paar als Klemmhalterung ausgebildet ist, mittels der ein jeweils eingebrachter Gegenstand darin klemmend halterbar ist.

Die EP 0 790 063 A 1 zeigt einen Gestell-Modul mit mehreren clipartigen Klemmhalterungen für die vertikale Halterung von Spritzenkörpern.

In der DE 40 21 836 C 1 wird eine Vorrichtung zur Handhabung von Spritzenzylindern und/oder Karpulen beschrieben, die aus mehreren komplexen Metallteilen, wie Träger- und Arretierungsplatte, bestehen, wobei die beiden Platten übereinander angeordnet sind und jeweils zueinander fluchtende Öffnungen zum klemmenden Aufnehmen der Spritzenzylinder bzw. Karpulen aufweisen.

Eine ähnliche Vorrichtung wird in DE 36 13 489 C 2 beschrieben.

Die vorgenannten Transportvorrichtungen weisen gravierende Nachteile auf. So sind sie nicht ohne weiteres für Primär-Packmittel aus Kunststoff oder Glas gleichermaßen geeignet, da die überwiegend verwendeten metallischen Materialien leicht zu Verkratzungen an Kunststoffkörpern führen können. Zum anderen sind sie auf Grund ihrer hohen Kosten, ihres hohen Gewichtes etc. nicht für die Einmalverwendung geeignet und müssen vor der Verwendung in Reinräumen aufwendig gereinigt werden. Während der Abfüllung von Fertigspritzen, die in Sterilform angeliefert werden, werden die leeren Behältnisse mit den vorgenannten Transportvorrichtungen in die Abfüllmaschine herangebracht. Zum Abfüllen, d.h. zum Einführen der Füllrohre in die Spritzen und zum nachfolgenden Einführen der Setzrohre in die mit Flüssigkeit gefüllten Spritzen, müssen diese in einem gesonderten Schritt geometrisch exakt ausgerichtet werden, da die Halterung von Hause aus diese Ausrichtung nicht gewährleistet. Dieses Ausrichten ist vor allem beim Setzen der Kolbenstopfen in die Spritzenkörper wichtig, da die Setzrohre vorzugsweise einen Durchmesser aufweisen, der nur geringfügig kleiner ist als der Innendurchmesser der Spritzenkörper.

In der WO 94/14484 wird ein-kammförmiger Halter speziell für Glasspritzen beschrieben, der ausschließlich für Glasspritzen mit Fingerauflage geeignet ist. Auch bei diesem Halter werden die Spritzen nicht zentriert, sondern hängen frei an ihrer Fingerauflage. Dies macht bei der Verarbeitung (z.B. dem Füllen, Verschließen etc.) das Herausnehmen der Spritzen aus der kammförmigen Einheit und eine Zentrierung notwendig.

Bei dem in der vorgenannten EP 0 790 063 A 1 zum Stand der Technik beschriebenen HYPAK® SCF®-System werden Kunststofflochplatten verwendet, in denen die Glasspritzen, an ihren Fingerauflagen hängend, lose gehalten werden. Bei diesem System ist daher ebenfalls ein gesonderter Zentrierungs-Vorgang notwendig.

Ein weiterer Nachteil dieser Halter mit frei an ihrer Fingerauflage hängenden Glasspritzen besteht darin, daß sich auch die Ausrichtung der Spritzen hinsichtlich der Fingerauflagen beim Transport ändert. Durch die relativ lose Halterung vibrieren ferner die Spritzenkörper beim Transport sehr stark, so daß es zur schmutzanziehenden statischen Aufladung kommt. Weiterhin können durch die starken Schüttelbewegungen der Spritzenkörper beim Transport Schmutzpartikel abgerieben werden und in die Spritzenkörper gelangen. Schließlich weisen voll besetzte Halter eine gewisse Durchbiegung auf, die sich insbesondere beim Füllvorgang nachteilig bemerkbar macht.

Der Erfindung liegt die Aufgabe zugrunde, die eingangs bezeichnete Transportvorrichtung für Packmittel für medizinisch wirksame Substanzen so auszubilden, daß sie insbesondere auf sehr einfache Weise herstellbar sowie universell verwendbar ist, und eine vibrations- bzw. rüttelfreie Halterung der Packmittel gewährleistet.

Die Lösung dieser Aufgabe gelingt ausgehend von der Transporteinrichtung für Packmittel für medizinisch wirksame Substanzen in Form einer biegestreifen Trägerplatte aus Kunststoff, die eine Vielzahl von Öffnungen zur Aufnahme der Behälter aufweist gemäß der Erfindung dadurch, daß die Trägerplatte aus einem geschäumten Kunststoff besteht und der freie Querschnitt der Öffnungen in Bezug auf den Querschnitt der Packmittel so abgestimmt und die Dicke der Trägerplatte so bemessen ist, daß die Packmittel jeweils in der Öffnung mit Klemmsitz zentriert fixiert aufnehmbar sind und auf ihnen keine Kratzspuren bei axialer Bewegung entstehen.

Die klemmende Halterung der Packmittel in den Öffnungen sorgt dafür, daß die Packmittel vibrations- und rüttelfrei transportierbar sind. Dadurch können einmal keine statischen Aufladungen entstehen, d.h. die Packmittel partikelfrei bleiben und zum anderen bleibt die Lage der Packmittel beim Transport unverändert, was sich vorteilhaft auf die Durchführung nachfolgender Arbeitsvorgänge auswirkt. Insbesondere ist keine nachträgliche Zentrierung der Packmittel beispielsweise für das Abfüllen notwendig, so daß die Abfüllung in der Transporteinrichtung erfolgen kann.

Die Trägerplatte ist ferner biegesteif und verursacht keine Kratzspuren auf der Behälterwand beim Einsetzen und Herausziehen der Behälter. Da die Trägerplatte aus einem geschäumten Kunststoff, vorzugsweise Polypropylen (PP) oder Polyester (PET), besteht, ist sie sehr kostengünstig herzustellen, wodurch die Trägerplatte ein wiederaufarbeitbarer Wegwerfartikel sein kann und eine kostenintensive Reinigung der Transportplatten entfällt. Wenngleich die geschäumte Trägerplatte von Haus aus bereits eine große Steifigkeit aufweist, so kann sie bei größeren Abmessungen versteifende Elemente aufweisen bzw. eine Rippenstruktur besitzen.

Die US 3, 643,812 zeigt eine Kunststoff-Box zum Aufbewahren von Prüfröhrchen. Sie weist neben einer aufgesetzten Gitterplatte, die die Röhrchen aufnimmt, eine Bodenplatte auf, die Vertiefungen zur Aufnahme der Röhrchenböden besitzt, damit sich die Röhrchen nicht seitlich verschieben können, sondern in aufrechter Lage fixiert sind. Dies bedeutet letztlich, daß die Röhrchen in der Gitterplatte nicht "zentriert klemmend fixiert" sind, wie im Anspruch 1 gekennzeichnet, sondern in den Aufnahmen der Gitterplatte durchaus beweglich sind. Letztlich liegt bei der bekannten Box ein Doppelebenen-Magazin wie im Fall der in der Beschreibung zitierten DE 36 13 489 A1 vor. Im Fall der Erfindung dagegen ist nur eine einzige Trägerplatte vorgesehen, in deren Öffnungen die Packmittel zentriert und durch Klemm-Kraft fixiert aufgenommen sind, so daß die Transportvorrichtung sehr einfach ist, da keine zweite, die vertikale Lage fixierende Platte notwendig ist.

Von der Gattung der Transporteinrichtungen für Packmittel zu unterscheiden sind die Mikrotiterplatten, wie sie beispielsweise durch die EP 0 976 453 A2 bekannt geworden sind. Diese Schrift zeigt eine Mikrotiterplatte für diagnostische Zwecke mit einer Vielzahl von in Zeilen und Spalten angeordneten Näpfchen mit einer Reagenz-Flüssigkeit, in die Proben eingelassen werden. Im Fall der vorgenannten Schrift sind dabei die Näpfchen separate Behälter, die in einem Grundgitter aufgenommen sind. Diese Mikrotiterplatte ist sozusagen ein "Standmodell", die einen festen Platz im Labor hat, und ist keine Transporteinrichtung im Sinne der Erfindung. Derartige Transporteinrichtungen werden in der Pharmaindustrie dazu benötigt, um Primär-Packmittel in großen Chargen, d.h. magaziniert, von Betrieb zu Betrieb und innerhalb der Betriebe zwischen einzelnen Stationen zwecks Durchführen von Bearbeitungsschritten zu transportieren.

Vorzugsweise ist gemäß einer Ausgestaltung der Erfindung die Oberfläche der geschäumten Trägerplatte porenfrei versiegelt. Dadurch ist es insbesondere möglich, die Trägerplatte mit den eingeschobenen Behältern im Autoklaven zu sterilisieren.

Die Öffnungen sind typischerweise in Reihen in der Trägerplatte ausgebildet. Um eine hohe Packungsdichte zu erreichen, ist dabei jeweils die benachbarte Reihe um den halben Lochabstand versetzt angeordnet.

Um einen sicheren Klemmsitz der Packmittel in der Trägerplatte zu erzielen, sind eine Reihe von Ausführungen denkbar.

Ein guter Klemmsitz ist beispielsweise erreichbar, wenn die Bohrungen konisch ausgebildet sind, mit der engsten Stelle am unteren Rand der Trägerplatte oder alternativ am oberen Rand der Trägerplatte.

Alternativ kann auch in der Öffnung jeweils ein umlaufender ringförmiger Fortsatz angeformt sein.

Ein derartiger Klemmsitz kann noch verbessert werden, wenn in der Öffnung jeweils zwei axial beabstandete umlaufende ringförmige Fortsätze angeformt sind.

Auch ist es gemäß einer Weiterbildung der Erfindung denkbar, daß in der Öffnung jeweils drei Kanten auf unterschiedlichen axialen Höhen für eine Dreipunktlagerung der Packmittel angeformt sind. Eine derartige Ausbildung verbessert den selbstzentrierenden Effekt und verhindert eine thermische Isolation eines bestimmten Behälterabschnittes.

Die sichere Führung der Packmittel hat darüber hinaus den Vorteil, daß es nicht durch Relativbewegungen zwischen Packmittel und Trägerplatte (Nest) zu statischen Aufladungen und zur Partikelbildung kommen kann.

Weiterhin verhindert die sichere Führung, daß die beim Transport unvermeidlichen Rüttelbewegungen dazu führen, daß nach oben hüpfende Packmittel die Siegelfolie, die zum Verschluß des gesamten Magazins dient, durchscheuern oder beschädigen, und damit die Gefahr von Unsterilitäten entsteht. Um dieses zu verhindern, werden beim Stand der Technik schützende Zwischenfolien oder Zwischenschäume eingesetzt, die durch die erfindungsgemäße Trägerplatte überflüssig werden.

Die Transportplatte kann, sofern sie diese Fixierung gewährleistet, auch zur direkten Befüllung der Packmittel benutzt werden, bei der dann im Gegensatz zur heutigen Praxis die Packmittel nicht mehr einzeln oder gruppenweise aus dem Nest gehoben und aufwendig zur Abfüllung zentriert und ausgerichtet werden müssen. Es können daher mit dem erfindungsgemäßen Nest gegenüber dem heutigen Stand der Technik erheblich höhere Abfülleistungen erzielt werden.

Weitere Ausgestaltungen der Erfindung sind in den Unteransprüchen gekennzeichnet und ergeben sich aus der Beschreibung von in den Zeichnungen dargestellten Ausführungsbeispielen.

Es zeigen:
- Fig. 1: in einer schematischen perspektivischen Darstellung den Grundaufbau der erfindungsgemäßen Trägerplatte für den Sammel-Transport von Primär-Packmitteln,
- Fig. 2: in vier Figurenteilen A - E Querschnittsansichten von fünf verschiedenen Ausbildungen des Klemmsitzes der Packmittel in Form von Spritzen mit Fingerauflagen in den Bohrungen der Trägerplatte, wobei die Fingerauflage übersteht,
- Fig. 3: in vier Figurenteilen A - D Querschnittsansichten von vier verschiedenen Ausbildungen des Klemmsitzes der Packmittel in Form von Spritzen mit Fingerauflage in den Bohrungen der Trägerplatte, wobei die Fingerauflage abgesenkt ist, und
- Fig. 4: eine schematische Draufsicht auf die mit Packmittel in Form von Spritzen mit Fingerauflage bestückte Trägerplatte, wobei die Fingerauflagen beidseitig parabelförmig auslaufen.
- Fig. 5: in vier Figurenteilen A - D Querschnittsansichten von vier verschiedenen Ausbildungen des formschlüssigen Klemmsitzes von Fläschchen in der Trägerplatte, und
- Fig. 6: ebenfalls in vier Figurenteilen A - D vier verschiedene Halterungen der Fläschchen nach Fig. 6 in Verbindung mit angeformten Lippen am Rand der Bohrungen.

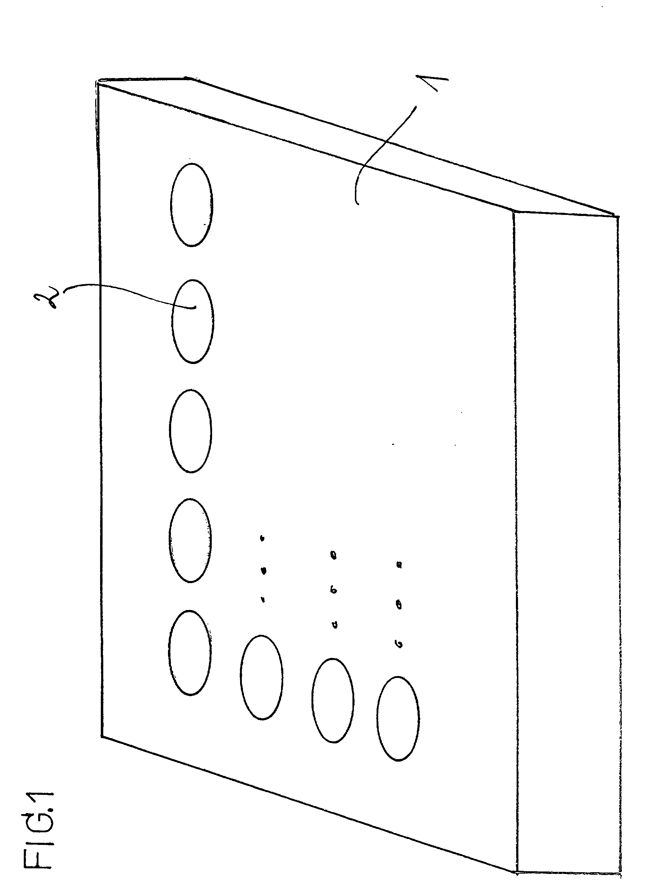

Die erfindungsgemäße Transportvorrichtung für Primär-Packmittel von medizinisch wirksamen Substanzen weist gemäß Fig. 1 eine einstückige biegesteife, rechteckförmige Trägerplatte 1 aus geschäumten Kunststoff, vorzugsweise geschäumten Polypropylen (PP) oder Polyester (PET) oder ähnlichen Materialien auf, wie sie beispielsweise in dem Messebericht zur Tokyo Pack 1992 in "Neue Verpackung", 12/92 Seiten 32 - 38 beschrieben werden, der hiermit durch Bezugnahme mit zum Offenbarungsinhalt gehören soll.

Die Abmessungen der Trägerplatte 1 richten sich nach den Anlagen, in denen die Transportplatte zum Einsatz kommt. Typischerweise hat die Rechteck-Trägerplatte eine Abmessung von etwa 20 cm X 25 cm, bei einer Dicke von ca. 1 - 2 cm. Zur Handhabung einer größeren Anzahl von Packmitteln können auch deutlich größere Trägerplatten bis zu Abmessungen von 100 x 100 cm bei einer Dicke von 2 - 3 cm zum Einsatz kommen.

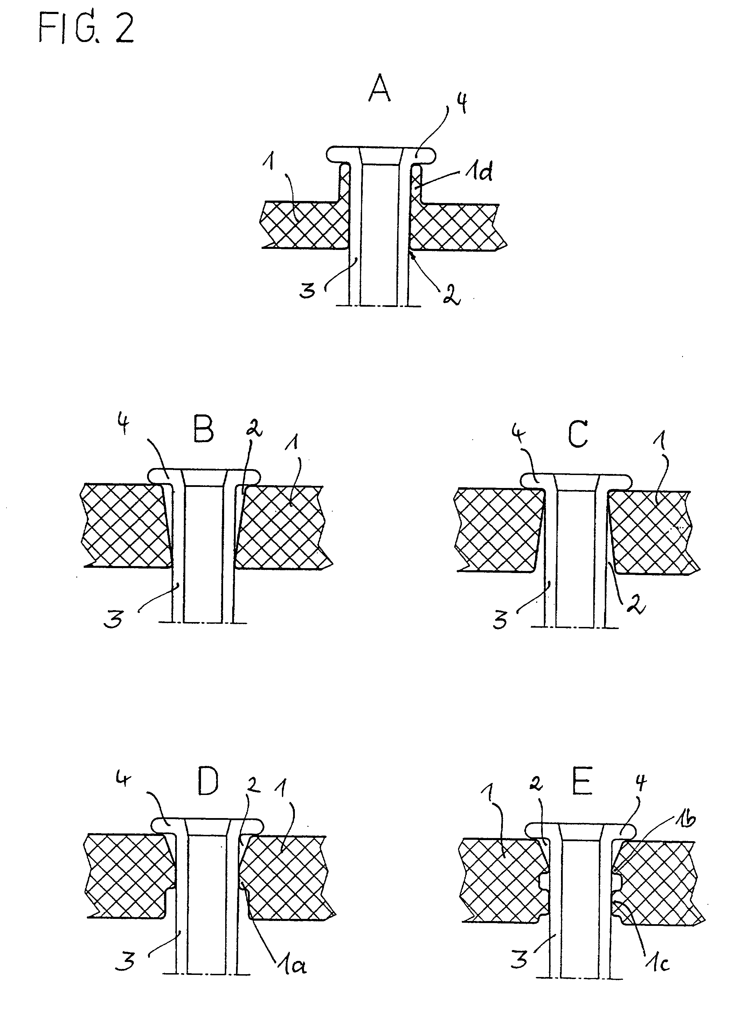

In der Trägerplatte 1 sind in mehreren Reihen, von denen nur eine komplett dargestellt ist, kreisrunde oder vieleckige Bohrungen 2 zur Aufnahme von Primär-Packmitteln 3 (Fig. 2 ff) mit zylinderförmiger Behälterwand ausgebildet. Diese Bohrungen 2 werden zweckmäßig bereits beim Herstellen der Trägerplatte ausgeformt. Sie können jedoch prinzipiell auch nachträglich durch ein entsprechendes Mehrfach-Bohrmodul ausgebohrt werden.

Ist der Packmittelquerschnitt nicht kreisrund, sondern z.B. dreieckig, dann weist die Bohrung 2 einen entsprechenden Querschnitt auf.

Vorzugsweise ist die Trägerplatte 1 so ausgebildet, daß sie eine porenfreie Oberfläche besitzt. Dies kann typischerweise durch eine nachträgliche Oberflächenbehandlung der geschäumten Trägerplatte geschehen.

Der Durchmesser der Bohrungen 2 ist so auf den Durchmesser der Packmittel 3 abgestimmt, daß die Packmittel klemmend und zentriert in der Bohrung aufgenommen sind. Dadurch verhindert die erfindungsgemäße Transportplatte Relativbewegungen zwischen Packmittel 3 und Trägerplatte 1 sicher und ist somit auch für verkratzungsempfindliche Packmittel, z.B. solche aus spröden Kunststoffen, geeignet.

Die erfindungsgemäße Transportplatte kann - wenn sie eine geschlossene porige Oberfläche aufweist - mit Vorteil zusammen mit leeren oder befüllten Packmitteln autoklaviert werden, so daß für diesen Sterilisierungsschritt kein "Umladen" der Packmittel notwendig ist.

Die Fig. 2 zeigt im Figurenteil A die Aufname eines Spritzenkörpers 3, der oben eine Figerauflage 4 aufweist, in der Trägerplatte 1, die um die Bohrung 2 herum einen umlaufenden Kragen 1d aufweist, auf dem die Fingerauflage, leicht abhebbar, aufliegt. Die Bohrung 2 ist dabei durchgehend zylindrisch.

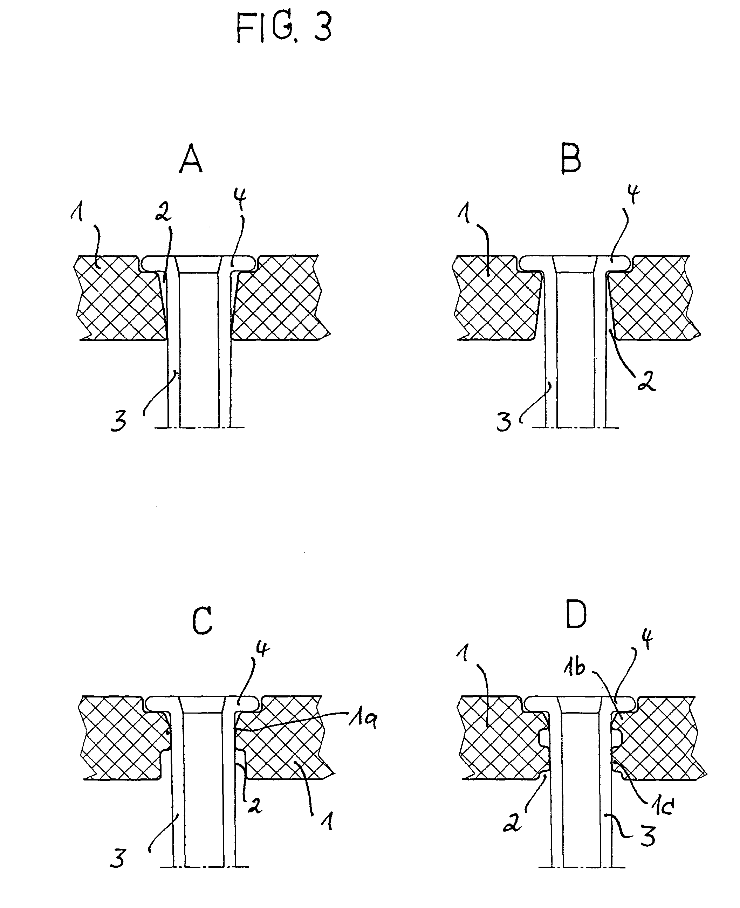

Wie in Fig. 2 in den Figurtenteilen B und C für Spritzenkörper 3, deren Fingerauflage 4 in der Bohrung 2 über die Trägerplatte 1 übersteht und in Fig. 3 in den entsprechenden Figurenteilen A und B für Spritzenkörper 3, deren Fingerauflage 4 bündig mit der Oberseite der Trägerplatte 1 abschließt, dargestellt ist, sind die Bohrungen 2 vorzugsweise konisch ausgeführt, so daß die eingesteckten Spritzenkörper 3 sicher fixiert und zentriert sind. So werden Toleranzen in der Spritzenkörpergeometrie ausgeglichen.

Dabei kann sich die konische Fläche nach unten (Fig. 2 B, Fig. 3 A) oder nach oben verjüngen (Fig. 2 C, Fig. 3 B).

Die Figuren 2 und 3 stellen dabei eine Querschnittansicht der Trägerplatte 1 in der Umgebung einer Bohrung 2 dar.

Das Packmittel 3 kann in der Öffnung 2 der Trägerplatte auch, wie in den Figuren 2 D und 3 C dargestellt, in der Weise klemmend gehalten werden, daß in der Öffnung 2 an der Trägerplatte 1 ein umlaufender ringförmiger Fortsatz 1 a angeformt ist, mit einem Durchmesser an seiner engsten Stelle, der etwas kleiner als der Durchmesser des Packmittels 3 ist.

Bei den Ausführungsformen nach den Figuren 2 E und 3 D sind dabei zwei axial beabstandete umlaufende ringförmige Fortsätze 1 b und 1 c angeformt, was den sicheren Halt noch verbessert.

In einer besonderen Ausführungsform der erfindungsgemäßen Transportplatte sind die Bohrungen 2 so gestaltet, daß die Führung und Klemmung der Packmittel 3 an drei Kanten (punktuelle Fortsätze) erfolgt, was den selbstzentrierenden Effekt verbessert, und eine thermische Isolation eines bestimmten Packmittelabschnittes verhindert. Diese Kanten befinden sich vorzugsweise auf unterschiedlichen axialen Höhen.

In einer besonderen Ausführungsform besteht die Transportplatte 1 aus einem solchen geschäumtem Kunststoff, der mit in Inertgasen (z.B. Stickstoff, Kohlendioxid, Argon) aufgeschäumt wurde. Dies verhindert sicher die Bildung von Ozongas bei der Sterilisation durch energiereiche Strahlung (γ-Strahlung oder Elektronenstrahlung).

Gemäß einer Ausgestaltung der Erfindung besitzt die Transportplatte aus geschäumtem Kunststoff versteifende Elemente.

In einer alternativen Ausführungsform weist die Transportplatte 1 eine Rippengeometrie zur Versteifung auf.

Vorzugsweise enthält die Transportplatte Löcher und Durchbrüche zur Verringerung des Ausströmungswiderstandes, was wichtig beim Arbeiten im Reinraum und beim Autoklavieren ist.

Die erfindungsgemäße Trägerplatte kann bei den unterschiedlichsten Handhabungsvorgängen beim Herstellen und Befüllen der Packmittel 3 eingesetzt werden. Sie ist auch als Einsatz in einem wannenförmigen Behälter als Transportverpackung für leere oder befüllte Packmittel 3 einsetzbar.

In einer besonderen Ausführungsform wird die Transportplatte 1 so stufig geformt, daß sich die Packmittelenden (in vertikaler Richtung gesehen) in unterschiedlicher Höhe befinden, so daß es z.B. bei Spritzen mit großen Fingerauflagen nicht zur Berührung benachbarter Fingerauflagen kommt.

In den Figuren 1 bis 3 ist eine einstückige Trägerplatte 1 dargestellt. Bei einer alternativen Ausführungsform besteht die Transportplatte aus mehreren Lagen, z.B. mindestens einer PE-Lage und Schaumlagen aus PP oder PET, wobei die Halterung durch die Schaumlagen erfolgt und die PE-Lagen zur Versteifung dienen. Anstelle von PE-Lagen können auch andere Kunststoff-Lagen, z.B. aus Polystyrol (PS), Polypropylen (PP) etc. eingesetzt werden.

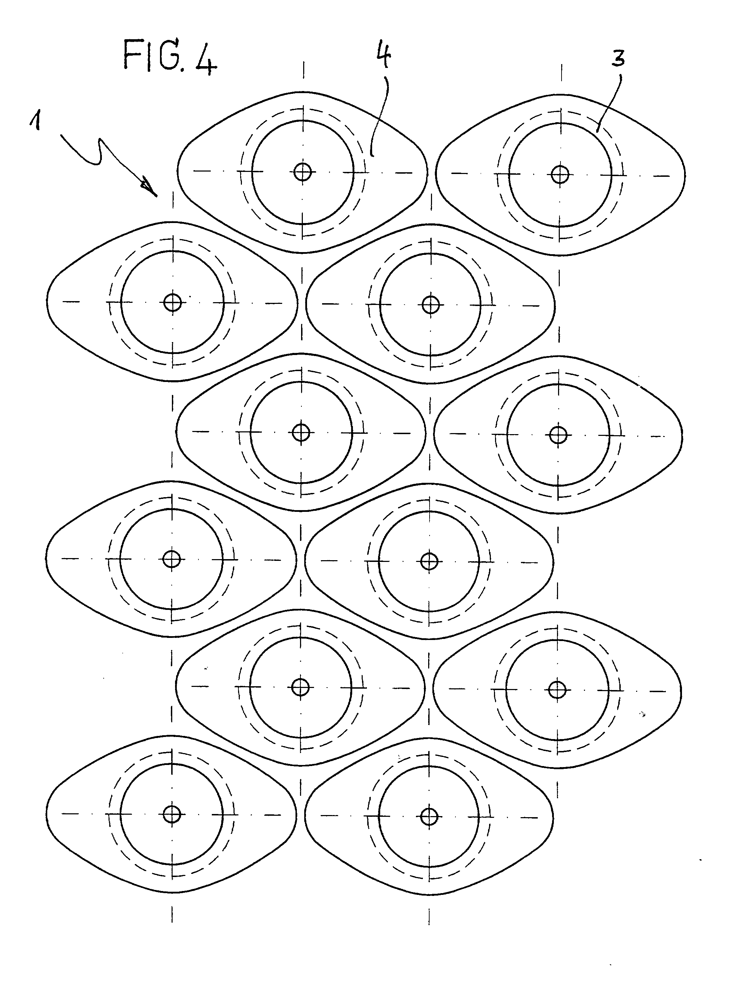

Die Figur 4 zeigt eine Ausbildung der Trägerplatte 1 mit mehreren, versetzt zueinander angeordneten Lochreihen, so daß bei Spritzen 3 mit einer besonders gestalteten, parabelförmigen Fingerauflage 4, eine sehr hohe Packungsdichte erreicht werden kann, was die Wirtschaftlichkeit bei der Herstellung und Befüllen der Spritzen erhöht und somit die Kosten senkt.

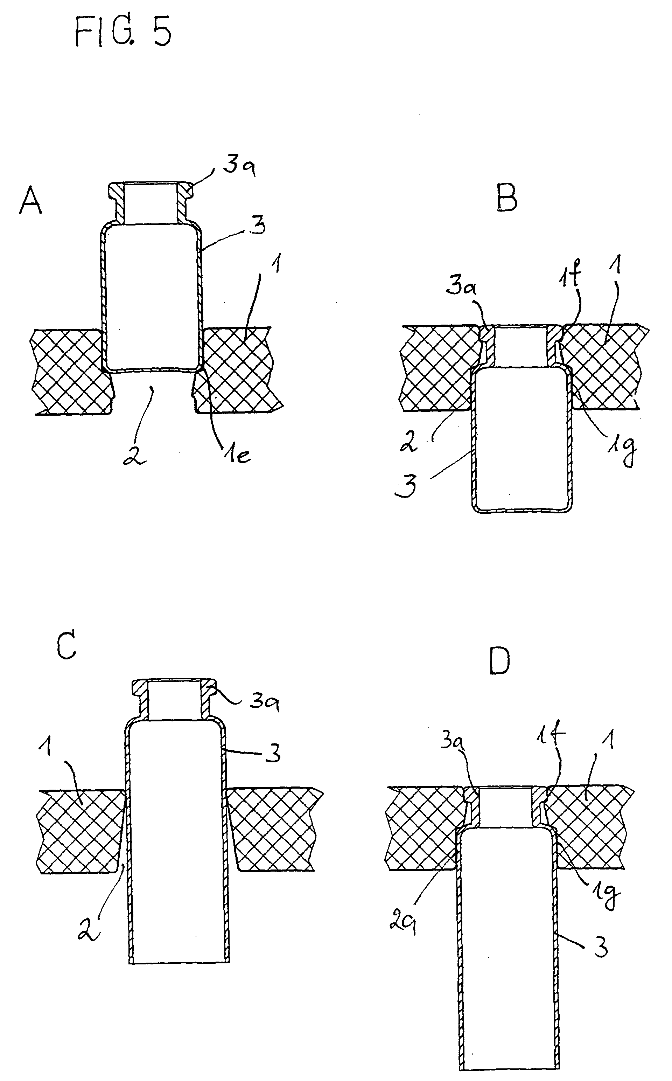

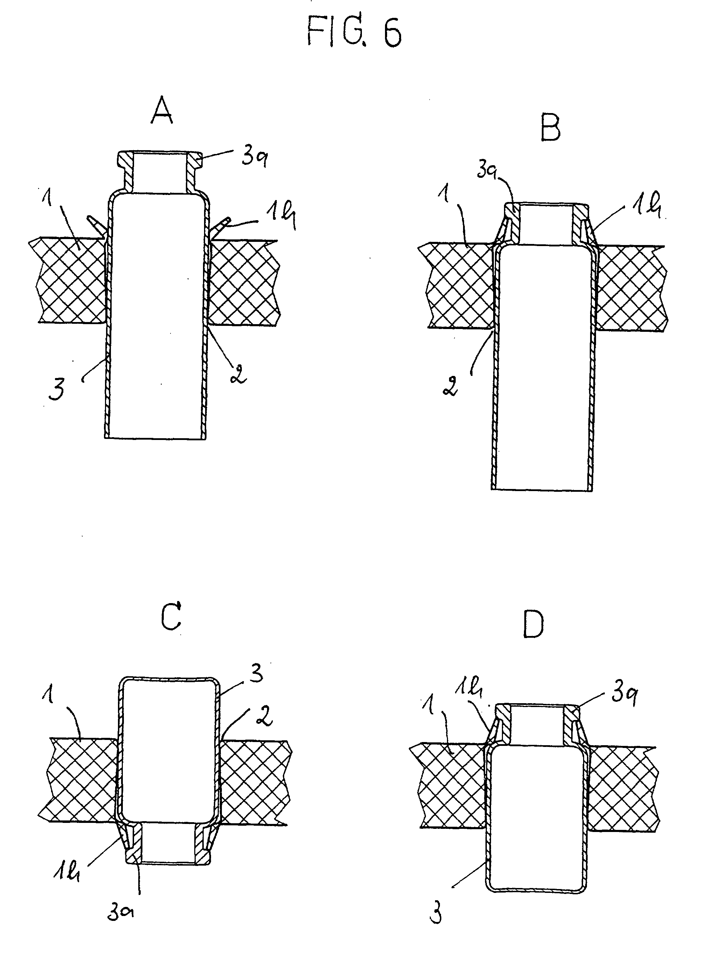

Die Figuren 2 bis 4 zeigen Ausführungsformen der Erfindung, bei denen als Packmittel 3 Spritzenkörper mit ihren Fingerauflagen 4 (Griffleisten) in der Trägerplatte 1 klemmend eingehängt sind. In den Figuren 5 und 6 sind verschiedene Möglichkeiten der Aufnahme von Packmitteln 3 in Form von unterschiedlich ausgebildeten Fläschchen in der Trägerplatte 1 dargestellt.

Der Figurenteil 5A zeigt eine stehende Halterung des Fläschchens 3, das verschlußseitig einen Randwulst 3a besitzt, in der Trägerplatte 1, wobei die Bohrung 2 einen entsprechenden Ansatz le für die Halterung des Fläschchenbodens besitzt.

Im Figurenteil 5B ist eine Halterung des gleichen Fläschchens dargestellt, bei der der Randwulst 3a in einem Absatz 1f in der Trägerplatte 1 bündig mit deren Oberseite aufgenommen ist, mit einer zusätzlichen formschlüssigen Halterung des oberen zylindrischen Abschnittes des Fläschchens in dem insoweit zylindrischen Abschnitt 1g der Bohrung 2. Das Fläschchen 3 wird bei der Halterung nach Fig. 5B von unten in die Bohrung 2 der Trägerplatte 1 eingeführt, bis der Randwulst 3a in den Absatz 1f einrastet. Es hängt dann in diesem Absatz und zwar kippsicher durch die klemmende Führung im Lochabschnitt 1g.

Im Figurenteil 5C wird ein etwas längeres Fläschchen 3 in einer konischen Bohrung 2 klemmend gehalten, vergleichbar mit der Halterung des Spritzenkörpers 3 in Fig. 2C.

Im Figurenteil D schließlich erfolgt die Halterung des längeren Fläschchens 3 analog der formschlüssigen Halterung in Fig. 5B über den Randwulst 3a im Absatz 1f.

In Fig. 6 sind vier weitere verschiedene Ausführungsformen für die Halterung von Fläschchen 3 in Bohrungen 2 der Trägerplatte 1 dargestellt, bei denen die Bohrungen 2, abgestimmt im Durchmesser auf den Durchmesser des Fläschchenmantels, durchgehend zylinderisch ausgebildet sind, mit mehreren, vorzugsweise drei an der Bohrung 2 angeformten flexiblen Lippen 1h.

Gemäß den Figurenteilen A und B wird das Fläschchen 3 von oben in die Bohrung 2 eingesteckt, bis die Lippen 1h an dem Hinterschnitt des Randwulstes 3a zum Anliegen kommen.

Der Figurenteil 6D zeigt für ein kürzeres, dickeres Fläschchen 3 die gleiche Halterung wie im Figurenteil B, d.h. das Fläschchen 3 wird mit den Lippen 1h am Hinterschnitt des Randwulstes 3a und mit dem Fläschchenmantel in die Bohrung klemmend geführt, gehalten. Der Figurenteil 6C zeigt eine ähnliche Über-Kopf-Halterung des Fläschchens 3.

Durch die Erfindung wird somit eine universelle Transportverpackung für Primär-Packmittel von medizinischen Substanzen, unabhängig von deren Querschnitt geschaffen, die folgende vorteilhafte Eigenschaften aufweist:
- Einfach, d.h. aus wenigen Teilen bestehend
- Fixierung und Zentrierung der Behälter unabhängig von Überständen (z.B. Fingerauflage bei Fertigspritzen), d.h. auch geeignet für Zylinderampullen, Ampullen, Fläschchen, Glaszylinder, die keine Überstände besitzen
- Ohne bewegte Teile
- Kostengünstig herzustellen
- Für den Einmalgebrauch geeignet
- Sterilisierbar, z.B. durch energiereiche Strahlung, sterilisierende Gase, z.B. Ethylenoxid oder Autoklavierung (121° C Wasserdampf)
- Gleichzeitiger Transportschutz
- Auch in Gefriertrocknungsanlagen einsetzbar.

## Patentansprüche

1. Transportvorrichtung für Packmittel für medizinisch wirksame Substanzen in Form einer biegesteifen Trägerplatte (1) aus Kunststoff, die eine Vielzahl von Öffnungen (2) zur Aufnahme der Packmittel (3) aufweist, **dadurch gekennzeichnet, daß** die Trägerplatte (1) aus einem geschäumten Kunststoff besteht und der freie Querschnitt der Öffnungen (2) in Bezug auf den Querschnitt der Packmittel (3) so abgestimmt und die Dicke der Trägerplatte (1) so bemessen ist, daß die Packmittel (3) jeweils in der Öffnung (2) mit Klemmsitz zentriert fixiert aufnehmbar sind und auf ihnen keine Kratzspuren bei axialer Bewegung entstehen.

2. Transportvorrichtung nach Anspruch 1, **dadurch gekennzeichnet, dass** der Kunststoff Polypropylen (PP) oder Polyester (PET) ist.

3. Transportvorrichtung nach Anspruch 2, **dadurch gekennzeichnet, dass** zumindest eine Oberfläche der Trägerplatte porenfrei versiegelt ist.

4. Transportvorrichtung nach Anspruch 2 oder 3, **dadurch gekennzeichnet, daß** die Trägerplatte (1) versteifende Elemente aufweist.

5. Transportvorrichtung nach einem der Ansprüche 2 bis 3, **dadurch gekennzeichnet, dass** die Trägerplatte (1) eine Rippengeometrie aufweist.

6. Transportvorrichtung nach einem der Ansprüche 1 bis 5, **dadurch gekennzeichnet, dass** die Öffnungen (2) in Reihen angeordnet sind und jeweils die benachbarte Reihe um den halben Lochabstand versetzt angeordnet ist.

7. Transportvorrichtung nach einem der Ansprüche 1 bis 6, **dadurch gekennzeichnet, dass** die Öffnungen (2) konisch ausgebildet sind, mit der engsten Stelle am unteren Rand der Trägerplatte (1) oder alternativ am oberen Rand der Trägerplatte (1).

8. Transportvorrichtung nach einem der Ansprüche 1 bis 6, **dadurch gekennzeichnet, dass** in der Öffnung (2) jeweils ein umlaufender ringförmiger Fortsatz (1a) angeformt ist.

9. Transportvorrichtung nach einem der Ansprüche 1 bis 6, **dadurch gekennzeichnet, dass** in der Öffnung (2) jeweils zwei axial beabstandete umlaufende ringförmige Fortsätze (1a, 1c) angeformt sind.

10. Transportvorrichtung nach einem der Ansprüche 1 bis 6, **dadurch gekennzeichnet, dass** in der Öffnung jeweils drei Kanten auf unterschiedlichen axialen Höhen für eine Dreipunktlagerung der Behälter (3) angeformt sind.

11. Transportvorrichtung nach einem der Ansprüche 1 bis 10, **dadurch gekennzeichnet, dass** die Trägerplatte (1) zusätzlich freie Öffnungen zur Verringerung des Anströmungswiderstandes aufweist.

12. Transportvorrichtung nach einem der Ansprüche 1 bis 11, **dadurch gekennzeichnet, daß** die Trägerplatte (1) gestuft ausgebildet ist.

13. Transportvorrichtung nach einem der Ansprüche 1 bis 12, **dadurch gekennzeichnet, dass** die Trägerplatte (1) einstückig oder mehrlagig ausgebildet ist.

## Claims

1. Transporting device for packaging articles for medically active substances, in the form of a flexurally rigid support plate (1) which is made of plastic and has a multiplicity of openings (2) for receiving the packaging articles (3), **characterized in that** the support plate (1) is made of a foamed plastic and the free cross section of the openings (2) is chosen in relation to the cross section of the packaging articles (3) in such a way, and the thickness of the support plate (1) is dimensioned in such a way, that the packaging articles (3) can be received in the respective opening (2) with a clamp fit and in a centred position and no scratch marks appear on them upon axial movement.

2. Transporting device according to Claim 1, **characterized in that** the plastic is polypropylene (PP) or polyester (PET).

3. Transporting device according to Claim 2, **characterized in that** at least one surface of the support plate is sealed off and free from pores.

4. Transporting device according to Claim 2 or 3, **characterized in that** the support plate (1) has stiffening elements.

5. Transporting device according to one of Claims 2 to 3, **characterized in that** the support plate (1) has a ribbed geometry.

6. Transporting device according to one of Claims 1 to 5, **characterized in that** the openings (2) are arranged in rows and the in each case adjacent row is staggered by half a hole distance.

7. Transporting device according to one of Claims 1 to 6, **characterized in that** the openings (2) are of conical shape, with the narrowest point at the bottom edge of the support plate (1) or, alternatively, at the top edge of the support plate (1).

8. Transporting device according to one of Claims 1 to 6, **characterized in that** a peripheral annular extension (1a) is integrally formed in the opening (2).

9. Transporting device according to one of Claims 1 to 6, **characterized in that** two peripheral annular extensions (1a, 1c) set axially apart from one another are integrally formed in the opening (2).

10. Transporting device according to one of Claims 1 to 6, **characterized in that** three edges are integrally formed in the opening at different axial heights for a three-point bearing of the containers (3).

11. Transporting device according to one of Claims 1 to 10, **characterized in that** the support plate (1) additionally has free openings for reducing the flow resistance.

12. Transporting device according to one of Claims 1 to 11, **characterized in that** the support plate (1) has a stepped configuration.

13. Transporting device according to one of Claims 1 to 12, **characterized in that** the support plate (1) is designed as one piece or as several layers.

## Revendications

1. Dispositif de transport de moyens d'emballage de substances actives médicinales sous la forme d'un plateau de support (1) rigide en flexion en plastique, qui présente une pluralité d'ouvertures (2) pour recevoir les moyens d'emballage (3), **caractérisé en ce que** le plateau de support (1) se compose d'un plastique moussé et la section transversale libre des ouvertures (2) par rapport à la section transversale des moyens d'emballage (3) est adaptée de telle sorte, et l'épaisseur du plateau de support (1) est dimensionnée de telle sorte, que les moyens d'emballage (3) puissent être reçus de manière fixe et centrée respectivement dans l'ouverture (2) avec un ajustement serré et qu'aucune rayure ne se produise sur eux lors d'un mouvement axial.

2. Dispositif de transport selon la revendication 1, **caractérisé en ce que** le plastique est du polypropylène (PP) ou du polyester (PET).

3. Dispositif de transport selon la revendication 2, **caractérisé en ce qu'**au moins une surface du plateau de support est scellée sans pores.

4. Dispositif de transport selon la revendication 2 ou 3, **caractérisé en ce que** le plateau de support (1) présente des éléments de renfort.

5. Dispositif de transport selon l'une quelconque des revendications 2 à 3, **caractérisé en ce que** le plateau de support (1) présente une géométrie nervurée.

6. Dispositif de transport selon l'une quelconque des revendications 1 à 5, **caractérisé en ce que** les ouvertures (2) sont disposées en rangées et la rangée respectivement voisine est décalée d'un demi-espace entre les trous.

7. Dispositif de transport selon l'une quelconque des revendications 1 à 6, **caractérisé en ce que** les ouvertures (2) sont réalisées avec une forme conique, avec le point le plus étroit au niveau du bord inférieur du plateau de support (1) ou en variante au niveau du bord supérieur du plateau de support (1).

8. Dispositif de transport selon l'une quelconque des revendications 1 à 6, **caractérisé en ce qu**'une saillie (1a) de forme annulaire périphérique est à chaque fois formée dans l'ouverture (2).

9. Dispositif de transport selon l'une quelconque des revendications 1 à 6, **caractérisé en ce que** deux saillies (1a, lc) de forme annulaire périphériques espacées axialement sont à chaque fois formées dans l'ouverture (2).

10. Dispositif de transport selon l'une quelconque des revendications 1 à 6, **caractérisé en ce que** trois arêtes sont formées à chaque fois à différentes hauteurs axiales dans l'ouverture pour un support à trois points des récipients (3).

11. Dispositif de transport selon l'une quelconque des revendications 1 à 10, **caractérisé en ce que** le plateau de support (1) présente en outre des ouvertures libres pour réduire la résistance à l'écoulement.

12. Dispositif de transport selon l'une quelconque des revendications 1 à 11, **caractérisé en ce que** le plateau de support (1) est réalisé avec des échelonnements.

13. Dispositif de transport selon l'une quelconque des revendications 1 à 12, **caractérisé en ce que** le plateau de support (1) est réalisé d'une seule pièce ou en plusieurs couches.
